# EUROPEAN PATENT APPLICATION

(11) **EP 3 815 606 A1**
(43) Date of publication of application: **05.05.2021**
(21) Application number: 19205586.1
(22) Date of filing: 28.10.2019
(51) Int. Cl.: A61B 5/06, A61B 5/00, G01R 33/00, G01R 33/28, A61B 8/00, A61M 25/00, H01L 27/08, H01L 29/93

(54) **A SENSING UNIT FOR MEASURING STIMULI IN A BODY**

(71) Applicant: Koninklijke Philips N.V., 5656 AG Eindhoven (NL)
(72) Inventor: VISSER, Cornelis Gerardus, 5656 AE Eindhoven (NL); DOODEMAN, Gerardus Johannes Nicolaas, 5656 AE Eindhoven (NL); KLEIJNEN, Mark Peter Paul, 5656 AE Eindhoven (NL); VAN PELT, Jeroen, 5656 AE Eindhoven (NL)
(74) Representative: Philips Intellectual Property & Standards

(57) **Abstract**

A sensing unit for use in-body comprises a variable impedance circuit for connection to the distal end of a transmission line and reflecting a carrier signal received from the transmission line. The variable impedance circuit comprises a variable impedance component having an impedance which varies non-linearly with applied voltage, a sensor for generating a voltage in response to a stimulus and a voltage bias system for creating a voltage bias for the variable impedance component. The voltage bias sets the operation point of the variable impedance component, such that the voltage changes from the sensor change the impedance of the variable impedance component non-linearly.

## Description

### FIELD OF THE INVENTION

This invention relates to a sensor circuit for measuring stimuli in a body and methods for operating the sensor circuit, and more specifically to the field of biased sensor circuits in a body.

### BACKGROUND OF THE INVENTION

Minimally invasive in-body procedures, such as guidewires, can be difficult to direct as they cannot be directly viewed.

A known way to obtain imaging information for a needle tip is to visualize the needle tip using an ultrasound PVDF (Polyvinylidene fluoride or polyvinylidene difluoride) sensor at the tip of the needle. This sensor is attached to the needle as part of a foil stack.

The PVDF sensor signal readout is done through interconnection with thin metal wires that run along the needle and a galvanic connection at the proximal side of the connector. Although the sensor signal is small, good signal-to-noise ratio (SNR) can be obtained by proper design of the readout circuit (e.g. a differential charge amplifier) and shielding. One solution to improve SNR is to use a lead zirconate titanate (PZT) sensor, which generates a much higher signal.

A problem with the above solution arises when used for guidewires. Long wires through the guidewire are complicated and expensive. The increased length can lead to signal degradation by increased capacitance and resistivity, which have a negative impact on the SNR. Signal integrity can be further affected by electromagnetic interference. Long wires through the guidewire are complicated in production and lead to additional costs. The connector for making electrical connection to the system is also a relatively expensive part, and hinders the clinical workflow.

In WO 2018/162361 a so-called single-wire RF resonator concept is disclosed to improve this situation. Here a varactor diode and a PVDF sensor are part of a resonating circuit. In the resonating mode, the voltage over the varactor diode is increased, leading to deeper modulation and better SNR. A readout is accomplished by using a single guidewire. Disadvantages include the fixed frequency and dimensions of such a resonator. These dimensions make integration in a guidewire complicated.

There is therefore a need for a sensing unit which can be easily integrated with a guidewire, which provides good SNR when integrated with a long guidewire and which can measure a variety of frequencies.

### SUMMARY OF THE INVENTION

The invention is defined by the claims.

According to examples in accordance with an aspect of the invention, there is provided a sensing unit for use in a body, comprising a variable impedance circuit for connection to a distal end of a transmission line, for reflecting a signal received from the transmission line, wherein the variable impedance circuit comprises:
a variable impedance component having an impedance which varies non-linearly with applied voltage;
a sensor for generating a voltage in response to a stimulus, for application to the variable impedance component, thereby to alter the impedance of the variable impedance component; and
a voltage bias system for creating a bias voltage for the variable impedance component for setting a voltage operating point of the variable impedance component.

The unit operates by reflecting a signal received from a transmission line. The signal reflects from the variable impedance circuit with an amplitude which is dependent on the impedance of the variable impedance circuit. This reflection can then be detected by an external detector. The variable impedance component has an impedance which changes in a non-linear way when a voltage is applied. For example, when a higher voltage is applied, the differential change in impedance is greater. The stimulus creates a voltage which modulates the impedance. The voltage bias system creates a voltage bias in the circuit and thereby sets the operating point of the circuit, such that a greater change in impedance of the variable impedance component is caused by the stimulus, thus giving improved signal to noise ratio.

The variable impedance component, the sensor and the voltage bias system may be connected in parallel.

The variable impedance component may comprise a diode, and the voltage bias system may comprise a DC voltage harvester for generating a DC voltage from the signal received from the transmission line. A diode can be used as a variable impedance component, as their impedance varies steeply with applied voltage just before the conduction band.

The DC voltage may be below the threshold voltage of the diode.

The stimulus may be an ultrasound signal, and the sensor may be piezoelectric material for detecting the ultrasound signal. This may, for example, be used for position detection of an interventional tool during an interventional procedure, using an ultrasound imaging field.

The stimulus may instead be an electromagnetic field and the sensor may be for detecting the electromagnetic field. The electromagnetic field may for example comprise microwaves, radio waves, infrared light, visible light, ultraviolet light or X-rays.

The stimulus may instead be a physiological signal generated by the body. Examples are temperature or pressure. Thus, the sensing unit may be used for in-body monitoring or it may be used for position detection.

The invention also provides a sensing system comprising:
a transmission line with a proximal end and a distal end;
a sensing unit at the distal end of the transmission line;
a signal generator at the proximal end of the transmission line; and
a signal detector for detecting the reflected signal from the variable impedance circuit. The signal detector is for example capacitively coupled to the transmission line.

The transmission line may be a guidewire. For example, if a sensor response to a stimulus needs to be measured inside a body, the sensing system can be attached to the end of the guidewire, and the guidewire used as the transmission line. In this example, no other equipment is needed on the guidewire to transmit the signal, thus reducing complexity in the measurement.

The invention also provides a sensing system comprising:
a transmission line with a proximal end and a distal end;
a sensing unit at the distal end of the transmission line;
a signal generator at the proximal end of the transmission line; and
a signal detector for detecting the reflected signal from the variable impedance circuit;
an external signal generator for generating a stimulus with known timing; and
a position detecting system for detecting the position of the sensing unit based on the timing of the stimulus and the timing of the detected reflected signal.

The external signal generator may be an ultrasound probe.

The invention also provides a method for sensing a stimulus in a body comprising:
receiving a signal at a sensor unit at a distal end of a transmission line;
creating a bias voltage for a variable impedance component having an impedance which varies non-linearly with applied voltage, thereby for setting a voltage operating point of the variable impedance component;
receiving a stimulus to be sensed at the sensor unit;
using the sensor unit to generate a voltage in response to the stimulus;
applying the generated voltage to the variable impedance component thereby to alter the impedance of the variable impedance component;
reflecting a signal received from the transmission line at the variable impedance component; and
detecting the reflected signal.

The stimulus may be a signal generated by the body (e.g. temperature or pressure) or it may be a stimulus delivered to the body, e.g. an imaging field.

Creating a bias voltage may comprise harvesting a DC voltage from the signal received from the transmission line.

The invention also provides a method for detecting the position of the sensing unit comprising:
generating the stimulus with known timing; and
detecting the position of the sensing unit based on the timing of the stimulus and the timing of the detected reflected signal.

The invention also provides a computer program comprising code means for implementing the generating of the stimulus and the detecting of the position of the sensing unit when said program is run on a processing system.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 shows a schematic representation of a sensing unit;
Fig. 2A and Fig. 2B show two possible circuit diagrams for the sensing unit attached to a guidewire, including a diode and a DC voltage harvester;
Fig. 3A, Fig. 3B and Fig. 3C show graphs demonstrating how the capacitance of a diode varies with a biased voltage;
Fig. 4A and Fig. 4B are graphs showing the reflected signal before and after being transferred over a transmission line;
Fig. 5A and Fig. 5B show a guidewire with the sensing unit at the distal end of the guidewire inside a lumen in a body, and an external signal generator outside the body;
Fig. 6A and Fig. 6B show a guidewire with the sensing unit used to find changes in local pressure inside a blood vessel;
Fig. 7A and Fig. 7B show a probe being used to find the depth of a needle inside a body;
Fig. 8A and Fig. 8B show a circuit and a schematic representation of a proof of concept test; and
Fig. 9A, Fig. 9B and Fig. 9C show graphs with the results of the proof of concept test.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a sensing unit for use in-body, comprising a variable impedance circuit for connection to the distal end of a transmission line and reflecting a carrier signal received from the transmission line. The variable impedance circuit comprises a variable impedance component having an impedance which varies non-linearly with applied voltage, a sensor for generating a voltage in response to a stimulus and a voltage bias system for creating a voltage bias for the variable impedance component. The voltage bias sets the operation point of the variable impedance component, such that the voltage changes from the sensor change the impedance of the variable impedance component non-linearly.

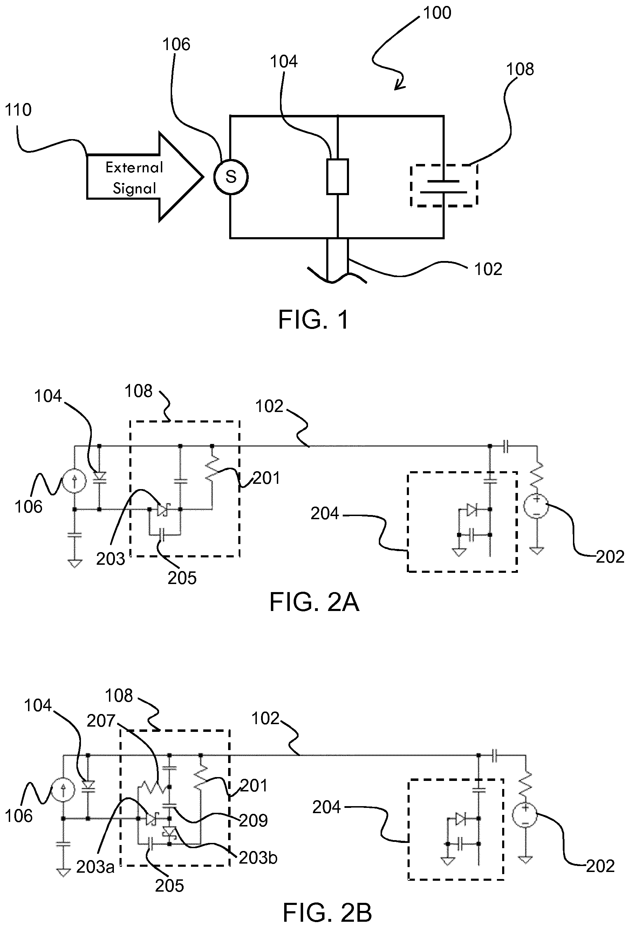

Fig. 1 shows a schematic representation of a sensing unit 100. It comprises a variable impedance component 104, a sensor 106 and a voltage bias system 108. There is a carrier signal delivered through a transmission line 102, which carrier signal is to be partially, or fully, reflected from the sensing unit 100. When the sensor 106 encounters a stimulus it creates an electric charge, which creates a voltage difference. The voltage bias system 108 also creates a voltage difference. The combination of the voltage differences is the total voltage that is applied to the variable impedance component 104.

In particular, the sensor 106 operates at the voltage imposed on it by the bias system 108, and it generates signal which is superposed on this operating voltage. For example it generates a charge which is stored on the self-capacitances of the variable impedance component, thereby changing the voltage across the variable impedance component and hence changing the impedance.

The applied voltage then changes the impedance of the component 104, which in turn changes the reflection coefficient of the sensing unit 100.

Fig. 2A and Fig. 2B show examples of circuit diagrams for the sensing unit attached to a transmission line 102, including a variable impedance component 104, such as a diode, and a voltage bias system 108, in this example a DC voltage harvester. A DC bias voltage is harvested from the carrier signal by the DC voltage harvester 108. The voltage harvester draws a small current and biases the diode 104 close to conduction. The carrier signal is transmitted by the transmission line 102 from a RF carrier signal source 202. The sensor 106, the diode 104 and the DC voltage harvester 108 are connected in parallel at the distal end of the transmission line 102.

The DC voltage harvester 108 produces a DC voltage, while the sensor 106 produces an AC voltage. The AC signal from the sensor 106 is superimposed on the DC signal.

Fig. 2A shows a DC voltage harvester 108 with a single harvester diode 203. The voltage harvester circuit is a so called Villard circuit. Over the harvester diode 203 a DC voltage appears, stored as a charge in the capacitor 205 parallel to the harvester diode. Via the resistor 201, this DC voltage is coupled to the diode 104.

Fig. 2B shows a so called Greinacher circuit. The DC voltage harvester circuit provides a voltage while there is no load current because the diode 104 is not in conductance. The Greinacher circuit charges the horizontal capacitor 205 at the bottom of the harvester. The vertical resistor 201 charges the diode 104 to a DC voltage, until conductance appears. The DC voltage harvester 108 circuit has a decoupling capacitor 209 to remove any DC signal from the carrier signal. The harvester diodes 203a and 203b rectify the AC signal to charge the output capacitor 205, creating a DC bias voltage. A high ohmic resistor 201 is used to reduce the current flow to the variable impedance component 104, ensuring the output capacitor 205 is not immediately discharged.

These and other voltage harvesting circuits will be known to those skilled in the art.

An ultrasound sensor can be used as the sensor 106, and positioned at the distal end of the transmission line 102 as part of an interventional device. This ultrasound sensor 106 is typically a piezoelectric material. The sensor material is a piezoelectric material such as PVDF (polyvinylidene fluoride or polyvinylidene difluoride, fluoroplymer material) and PZT (lead zirconate titanate, inorganic material).

The signal is transferred over the transmission line 102 by modulating a carrier signal via modulated backscatter, in other words, reflecting the carrier signal. Backscatter modulation is performed by a variable capacitance at the wave reflection position. The variable impedance component 104 can be a diode, positioned at or near the piezoelectric material and electrically connected with the ultrasound sensor 106. The diode 104 is added for its voltage-dependent capacitive properties. The biasing improves the deepness of the amplitude modulation by the sensing unit 100.

A detector 204 can then be used to read the reflected signal from the sensing unit 100. Since the carrier signal and the reflected signal will both be read by the detector 204, some processing of the detected signal is needed to read only the reflected signal. This could be done by applying an advanced synchronous detector with additional coupling the signal generator 202 and the detector 204, such that the detector 204 is aware of the signal from the signal generator 202 and can subtract it from the overall signal it detects. The subtraction may be done by a processor. The detector 204 may comprise a galvanic connector to detect the signal.

This carrier signal can have a broad frequency range. Different transmission lines 102 may have different properties, such as attenuation and signal velocity, for different frequencies. The carrier signal can also be one of many signal types, such as a repeating pulse, sine waves, square waves and any combination thereof. The carrier signal frequency and type can be dynamically chosen for optimal performance.

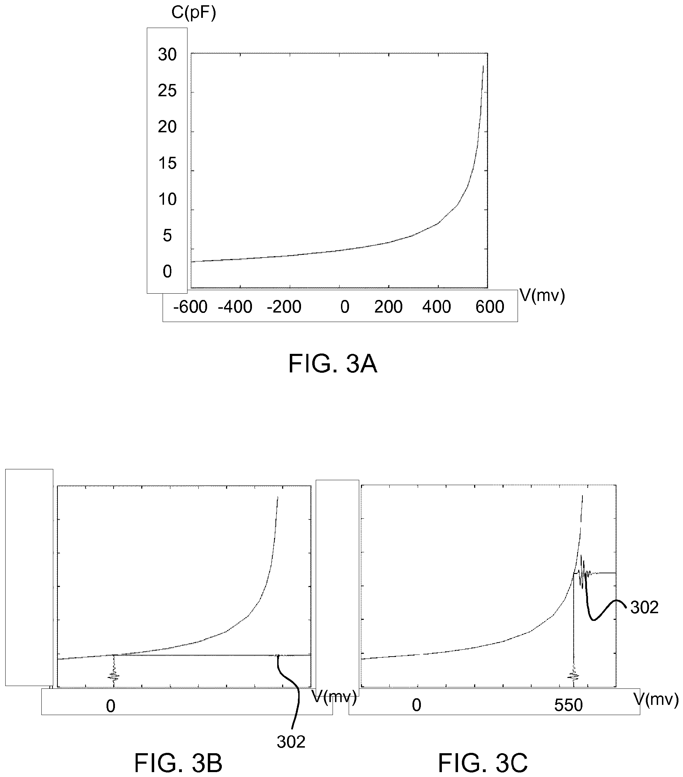

Fig. 3A, Fig. 3B and Fig. 3C show graphs demonstrating how the capacitance of a diode 104 varies with a biased voltage.

Fig. 3A shows the capacitance as function of bias voltage.

When a stimulus 110 reaches the sensor 106, an electrical charge is generated and the sensor 106 acts as a voltage source with an amplitude smaller than the diode threshold voltage. This voltage peak is fed to the diode 104, resulting in a short duration change in capacitance. The change in capacitance depends on the magnitude of the voltage peak and the characteristics of the diode 104.

In Fig. 3B there is no bias voltage, and the signal is received as a fluctuation at 0V. Since the voltage peak from the sensor 106 is very small, the change in capacitance (dC/dV) is relatively small, resulting in a reflected signal 302 with a small amplitude and a poor SNR.

A so-called single-wire RF resonator concept was disclosed in WO 2018/162361 to improve this situation. Here the diode 104 and sensor 106 are part of a resonating circuit. In the resonating mode the voltage over the diode is increased, leading to deeper modulation and better SNR. This only works for a chosen resonating frequency which the circuit is built for.

A recent insight suggests that the steepness of the diode capacitance curve increases strongly when applying a bias near the threshold voltage (where the diode 104 starts conducting). In Fig. 3A, dC/dV steepness increases significantly at a bias voltage of >0.4 Volts where the diode 104 starts conducting.

Fig. 3C shows how a small variation in voltage over the diode 104 can generate a much larger reflected signal 302 at the steep part of the curve, when a bias of 550mV is applied. This results in an increased modulation depth and a larger amplitude of the reflected signal 302 which leads to a better SNR.

The operating point of the diode 104 is pushed into the direction of the threshold voltage, but not into conduction. A small leakage current of < 50 µA is acceptable and contributes to equilibrium with a high-ohmic voltage source.

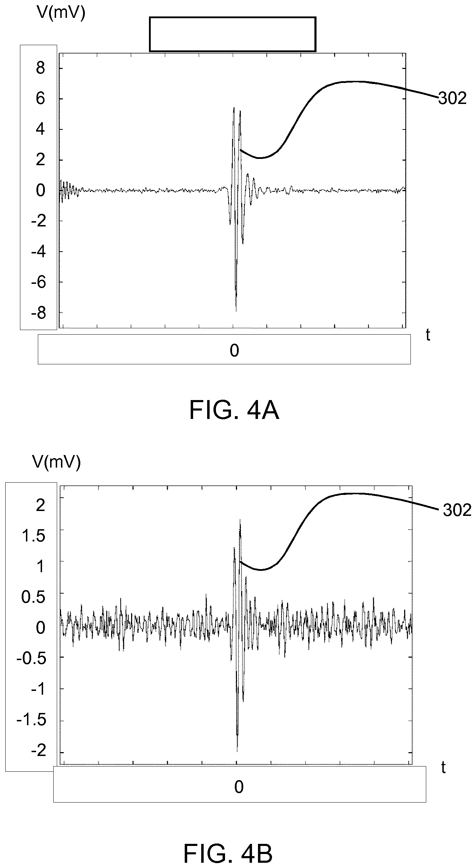

Fig. 4A and Fig. 4B are graphs showing a typical reflected signal 302 before and after being transferred over a transmission line 102. As can be seen in Fig. 4A, the reflected signal 302 directly form the sensing unit 100, before being transferred over a transmission line 102, is relatively free of electrical noise. Fig. 4B, however, shows that the reflected signal 302 after being carried by a transmission line 102, demodulated by the detector 204 and amplified to compensate for transmission losses, does have a relatively large amount of electrical noise. This may be due to many factors, including thermal noise in the transmission line 102 and electromagnetic interference from electrical components nearby. The electrical noise makes detecting signals with smaller amplitude difficult, and reduces the accuracy of the signals which can be distinguished from the noise. The transmission line 102 also attenuates the signal (reduces the amplitude over length) and is dependent on the length and the electrical properties of the transmission line 102. As can be seen in Fig. 4B, the peak of the reflected signal 302 has a smaller value than the peak before the reflected signal 302 has travelled through the transmission line 102, shown in Fig. 4A. Therefore a reflected signal 302 with a larger amplitude is needed for better SNR. This can be achieved due to the voltage bias system 108 allowing much larger changes in impedance of the variable impedance component 104.

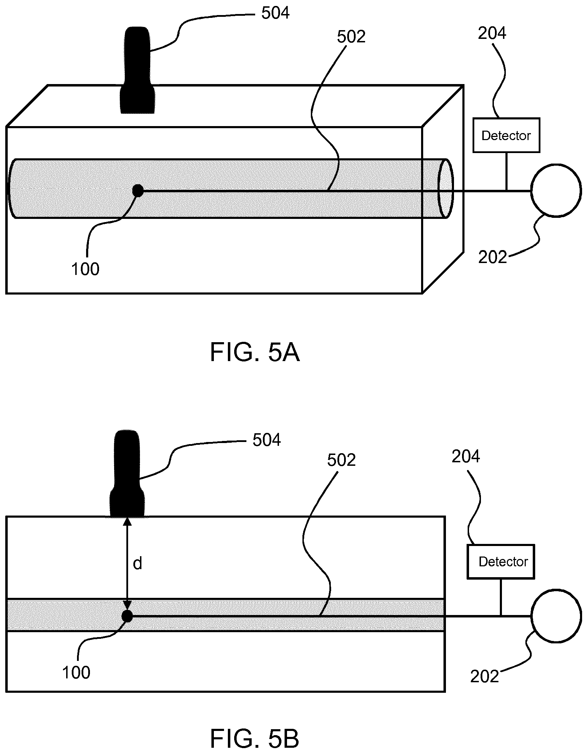

Fig. 5A and Fig. 5B show a guidewire 502, with the sensing unit 100 at the distal end of the guidewire 502, inside a lumen in a body, and an external signal generator 504 such as a probe, outside the body.

A carrier signal is fed to the guidewire 502 that acts as a transmission line 102. Since the guidewire 502 is used as the transmission line 102, no wires are needed through the interventional device. Since the impedance of the sensing unit 100 is not matched to the guidewire 502 impedance, at least part of the carrier signal is reflected.

When the sensor 106 in the sensing unit 100 receives a stimulus 110 from the probe 504, it generates a small charge that changes the impedance of the sensing unit 100, due to the variable impedance component 104. This change in impedance of the sensing unit 100 results in a change in reflected signal 302 amplitude. The change in reflection generates amplitude modulation side bands. The reflected signal 302 can be distinguished at the proximal end of the guidewire 502 from the carrier signal by a processor in the detector 204. The reflected signal 302 contains information on the signal created by the sensor 106. The sensor signal is converted into a backscattered phase modulated signal, which is much less sensitive to signal degradation and interference.

The stimulus 110 could, for example, be a pulsed field, where the characteristics of the pulsed field are such that the pulse has a sufficiently short duration to allow a defined measurement time to be identified. The pulse may be repeated regularly, where the pulse repetition rate allows multiple measurements in a short time.

The pulsed field could be acoustic pulses, such as ultrasound pulses, or of electromagnetic pulses, such as X-ray pulses.

The sensing unit 100 is provided to respond to a stimulus 110 having known timing properties (i.e. the timing of a pulse is known) in order to modulate the signal in the guidewire 502, and thus 'encode' timing information into the reflected signal 302.

The distance between the sensing unit 100, and therefore the distal end of the guidewire 502, and the probe 504, d, can be calculated from the timing difference between the stimulus 110 being emitted and the reflected signal 302 being measured at the detector 204, as well as from information of the velocity at which the stimulus 110 travels through the body. The time difference between the stimuli 110 being emitted and the sensing unit 100 reflecting the carrier signal is the time it takes for the stimulus 110 to travel the distance d. If the velocity of the stimulus 110 travelling through the body is known, the distance d can be calculated. Since the timing information of when the carrier signal is reflected is not known, it might also be necessary to know how long it takes for the reflected signal 302 to be transmitted to the detector 204 from the sensing unit 100 through the guidewire 502. This time is then subtracted from the time difference of the stimuli 110 being emitted and the reflected signal 302 being detected, in order to have an accurate time of the stimulus 110 travelling though the body.

The time it takes for the reflected signal to travel from the sensing unit 100 to the detector 204 can be obtained through pre-calibration of the guidewire 502 with different carrier signal frequencies. This is only necessary when the time for the stimulus 110 to travel through the body is of similar magnitude to the time taken by the reflected signal 302 to travel from the sensing unit 100 to the detector 204 and be processed.

It may also be possible to include many sensing units 100 on a guidewire 502, simultaneously sensing different stimuli 110, such as different frequencies of ultrasound waves, or different stimuli 110 types, such as ultrasound waves and electromagnetic waves. This may be achieved by having various frequencies in the carrier signal, and preparing the sensing units 100 such that each sensing unit 100 reflects a different frequency, or set of frequencies. In this way, the reflected signal 302 can be separated by frequencies, or set of frequencies, and the signal from each sensing unit 100 processed separately. The sensing units could be placed at different known distances on the guidewire.

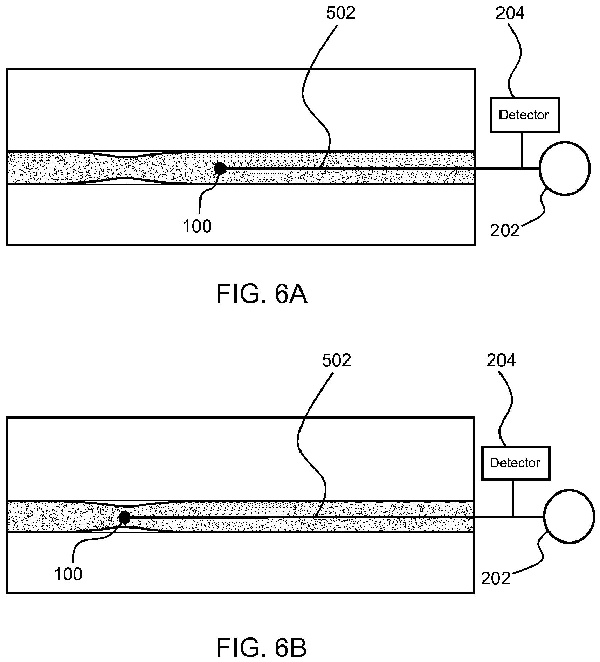

Fig. 6A and Fig. 6B show a guidewire 502 with a sensing unit 100 used to find changes in local pressure inside a blood vessel. In this case the sensing unit 100 may be prepared to sense local blood pressure. If the local blood pressure suddenly changes, it might indicate a local blockage in the blood vessel.

It is also possible to have a sensing unit 100 which gives other in-body physiological signals, such as temperature, heart rate or oxygen levels. It may also be possible to include many sensing units 100 on a guidewire 502, sensing different physiological signals at once.

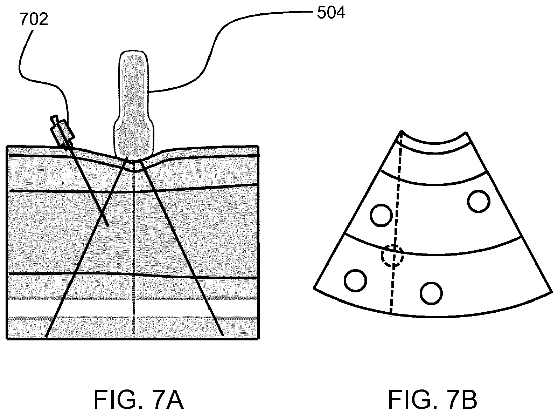

Fig. 7A and Fig. 7B show a probe 504 being used to find the depth of a needle 702 inside a body. The sensing unit 100 can be integrated into the distal end of the needle 702 in order to find the depth of the needle tip 702 in a body. An ultrasound probe 504 emits ultrasound acoustic waves which travel through the body. When the sensing unit 100 on the needle 702 senses the ultrasound waves it reflects a carrier wave travelling through either the needle 702 or wires in the needle 702. The reflected wave 302 can then be measured at the proximal end of the needle 702. The time taken between the probe 504 emitting the ultrasound wave and the carrier wave being reflected is the time that the ultrasound wave has travelled through the body. Using the knowledge of how fast an ultrasound acoustic wave can travel through a body, the distance between the probe 504 and the sensing unit 100 can be found, and therefore we can know how deep inside the body the needle 702 is.

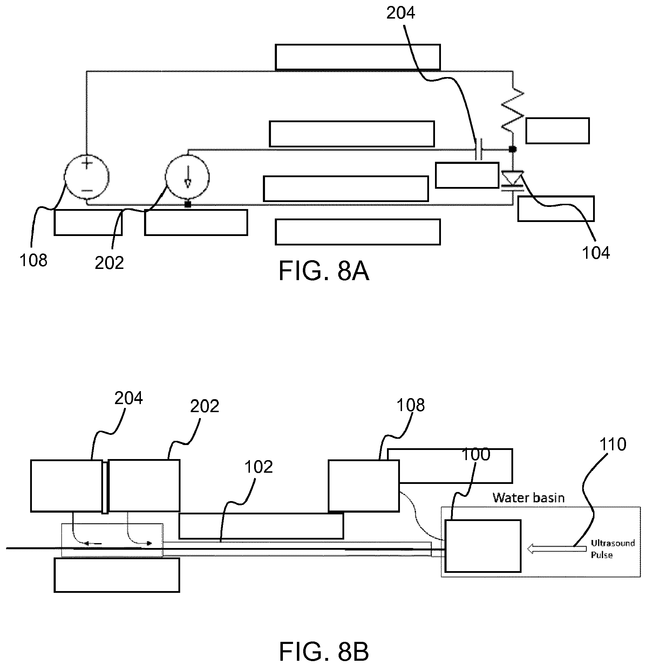

Fig. 8A and Fig. 8B show a circuit and a schematic representation of a proof of concept test. Some preliminary tests were done to demonstrate the biased diode concept. For this demonstration the variable impedance component 104 was a varactor diode and was biased with an external voltage source 108 (a conventional lab power supply). The bias wire was shielded to avoid external signal pick-up. An RF carrier of 743 MHz was used.

Fig. 8A shows the circuit used to show that the amplitude of the reflected signal 302 from a diode 104 is larger when a voltage bias is applied. In Fig. 8B the sensing unit 100 was placed in a water basin and an ultrasound pulse 110 was transmitted through the water.

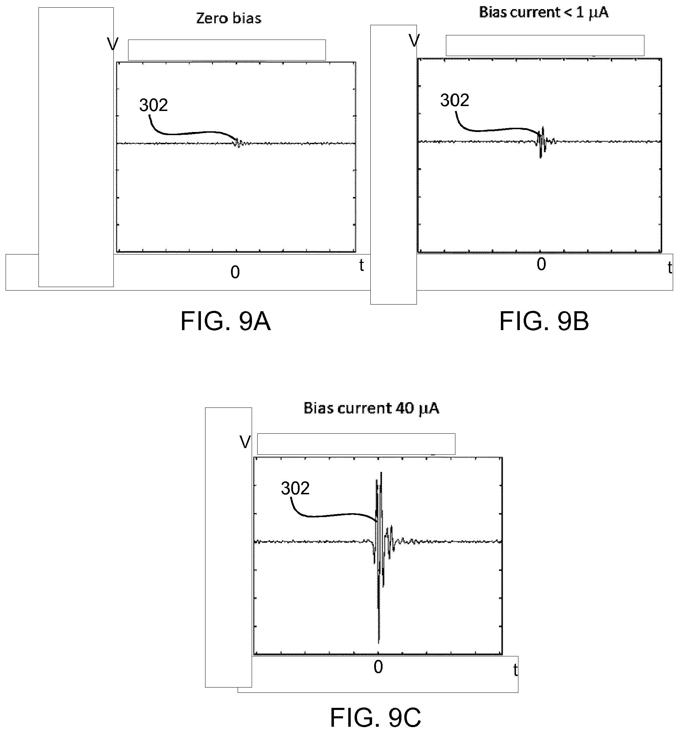

Fig. 9A, Fig. 9B and Fig. 9C show graphs with the results of the proof of concept test. Fig. 9A shows the reflected signal 302 when no current bias was applied. Fig. 9B shows that the reflected signal 302 has a larger amplitude even when a bias current of < 1 µA was applied, and Fig. 9C shows an even larger amplitude of the reflected signal 302 with a bias current of 40 µA. Although the noise chain of the set-up was not characterized and significant noise contribution from the bias power supply 108 was encountered, these results clearly demonstrate the positive effect of using a bias on the SNR.

A varactor diode may be used as the variable impedance component 104. It is also possible to use any diode operating close to its conduction band (e.g. Schottky, junction, varactor and other diode types). Diodes can be chosen for optimal junction capacitance and high frequency properties.

The components of the sensing unit 100 can be integrated in an application-specific integrated circuit (ASIC). An ASIC would make integration of the sensing unit 100 with a guidewire 502 relatively simple.

It is also possible to combine this concept with wireless powering and signal readout. No galvanic connector would be needed and the reflected signal 302 readout can be done through a capacitive coupler. Wireless powering and readout can simplify the usage of the system as it avoids having components at the proximal end of the system, where the system is controlled.

The RF frequency of the RF generator 202 will be selected taking various factors into account. Because the transmission line (e.g. guidewire) does not have a constant and well-defined impedance at the position of the RF generator 202 on the guidewire, there will be a mismatch between the fixed impedance of RF generator 202 and the guidewire impedance. The guidewire impedance at this point depends, among other things on the RF frequency.

Some RF frequencies will give a better efficiency and other RF frequencies a very poor efficiency in transferring the RF carrier from the RF generator to the guidewire. To have the proper RF carrier level injected to the guide wire, there are various options.

A first option is to use a frequency where the efficiency is high. In this case, a frequency sweep can be applied and the amplitude of the returned modulated signal is a measure for finding this optimal frequency.

A second option is to use a variable impedance transforming network, adapting the variable guide wire impedance to the RF generator impedance. This is known as an automatic antenna (impedance) tuner.

A third option is to increase or decrease the RF generator signal amplitude to the requested RF signal level at the guidewire. In case of an impedance mismatch, only port of the RF signal will be transferred to the guidewire and the other part will be dissipated in the RF generator.

The system may make use of a processor to perform the data processing. The processor can be implemented in numerous ways, with software and/or hardware, to perform the various functions required. The processor typically employs one or more microprocessors that may be programmed using software (e.g., microcode) to perform the required functions. The processor may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application specific integrated circuits (ASICs), and field-programmable gate arrays (FPGAs).

In various implementations, the processor may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measures cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". Any reference signs in the claims should not be construed as limiting the scope.

## Claims

1. A sensing unit (100) for use in a body, comprising a variable impedance circuit for connection to a distal end of a transmission line (102), for reflecting a signal received from the transmission line (102), wherein the variable impedance circuit comprises:
a variable impedance component (104) having an impedance which varies non-linearly with applied voltage;
a sensor (106) for generating a voltage in response to a stimulus (110), for application to the variable impedance component (104), thereby to alter the impedance of the variable impedance component (104); and
a voltage bias system (108) for creating a bias voltage for the variable impedance component (104) for setting a voltage operating point of the variable impedance component (104).

2. A sensing unit (100) as claimed in claim 1, wherein the variable impedance component (104), the sensor (106) and the voltage bias system (108) are connected in parallel.

3. A sensing unit (100) as claimed in claim 2, wherein the variable impedance component (104) comprises a diode, and the voltage bias system (108) comprises a DC voltage harvester for generating a DC voltage from the carrier signal received from the transmission line (102).

4. A sensing unit (100) as claimed in claim 3, wherein the DC voltage is below the threshold voltage of the diode.

5. A sensing unit (100) as claimed in any one of claims 1 to 4, wherein the stimulus (110) is an ultrasound signal, and the sensor (106) is a piezoelectric material for detecting the ultrasound signal.

6. A sensing unit (100) as claimed in any one of claim 1 to 4, wherein the stimulus (110) is an electromagnetic field and the sensor (106) is for detecting the electromagnetic field.

7. A sensing unit (100) as claimed in any one of claims 1 to 4, wherein the stimulus (110) is a physiological signal generated by the body.

8. A sensing system, comprising:
a transmission line (102) with a proximal end and a distal end;
a sensing unit (100) as claimed in any one of claims 1 to 7 at the distal end of the transmission line (102);
a signal generator (202) at the proximal end of the transmission line (102); and
a signal detector (204) for detecting the reflected signal (302) from the variable impedance circuit.

9. A sensing system as claimed in claim 8, wherein the transmission line (102) is a guidewire (502).

10. A sensing system, comprising:
a transmission line (102) with a proximal end and a distal end;
a sensing unit (100) as claimed in any one of claims 1 to 6 at the distal end of the transmission line (102);
a signal generator (202) at the proximal end of the transmission line (102); and
a signal detector (204) for detecting the reflected signal (302) from the variable impedance circuit;
an external signal generator (504) for generating the stimulus (110) with known timing; and
a position detecting system for detecting the position of the sensing unit (100) based on the timing of the stimulus (110) and the timing of the detected reflected signal (302).

11. A sensing system as claimed in claim 10, wherein the external signal generator (504) is an ultrasound probe.

12. A sensing method, comprising:
receiving a signal at a sensor unit (100) at a distal end of a transmission line (102);
creating a bias voltage for a variable impedance component (104) having an impedance which varies non-linearly with applied voltage, thereby for setting a voltage operating point of the variable impedance component (104);
receiving a stimulus to be sensed at the sensor unit (106);
using the sensor unit (106) to generate a voltage in response to the stimulus (110);
applying the generated voltage to the variable impedance component (104) thereby to alter the impedance of the variable impedance component (104);
reflecting a signal received from the transmission line (102) at the variable impedance component (104); and
detecting the reflected signal (302).

13. A sensing method as claimed in claim 12, wherein creating a bias voltage comprises harvesting a DC voltage from the carrier signal received from the transmission line (102).

14. A method as claimed in claim 12 or 13, comprising:
generating the stimulus (110) with known timing; and
detecting the position of the sensing unit (100) based on the timing of the stimulus (110) and the timing of the detected reflected signal (302).

15. A computer program comprising code means for implementing the generating of the stimulus (110) and the detecting of the position of the sensing unit (100), in the method of claim 14, when said program is run on a processing system.
